Europäisches Patentamt

European Patent Office

Office européen des brevets

(19)

(11) Publication number: **0 080 384**
**B1**

(12) **EUROPEAN PATENT SPECIFICATION**

(45) Date of publication of patent specification: **28.08.85**

(51) Int. Cl.⁴: **C 12 N 5/02**

(21) Application number: **82306271.6**

(22) Date of filing: **24.11.82**

(54) A bed for culturing biological cells and a culture method employing said bed.

(30) Priority: **24.11.81 JP 186947/81**

(43) Date of publication of application:
**01.06.83 Bulletin 83/22**

(45) Publication of the grant of the patent:
**28.08.85 Bulletin 85/35**

(84) Designated Contracting States:
**DE FR GB**

(56) References cited:
**DE-A-3 035 118**
**FR-A-2 311 845**
**US-A-4 024 020**

**CHEMICAL ABSTRACTS, vol. 84, 1976, page 57, no. 136782y, Columbus Ohio (USA); T. MATSUDA et al.: "Modification and characterization of polystyrene surface used for cell culture"**

**CHEMICAL ABSTRACTS, vol. 81, 1974, page 4, no. 64028v, Columbus Ohio (USA); T. MATSUDA et al.: "Modification and characterization of polystyrene surfaces used for cell culture"**

**SCIENCE, vol. 178, 6th October 1972, pages 65-67; R.A. KNAZEK et al.: "Cell culture on artificial capillaries: An approach to tissue growth in vitro"**

(73) Proprietor: **JAPAN SYNTHETIC RUBBER CO., LTD.**
**11-24, Tsukiji-2-chome Chuo-ku**
**Tokyo (JP)**

(73) Proprietor: **RIKAGAKU KENKYUSHO**
**2-1 Hirosawa**
**Wako-shi Saitama-ken (JP)**

(72) Inventor: **Takamatsu, Toshiaki**
**20-5, Asagayaminami-1-chome**
**Suginami-ku Tokyo (JP)**
Inventor: **Kaneko, Ichiro**
**428, Kamishakujii-1-chome**
**Nerima-ku Tokyo (JP)**
Inventor: **Kariya, Masao Nihongoseigomu-Shataku**
**2-104, 29, Aobadai-2-chome Midori-ku**
**Yokohama-shi Kanagawa-ken (JP)**

(74) Representative: **Tubby, David George et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to a method for culturing cells, which may be derived from mammals or other organisms, and to a method of culturing these cells employing the bed.

Considerable research has been carried out in recent years to investigate the reactions and other characteristics of cells of many organisms under various conditions and the manufacture of products arising from the culture of such cells. In particular, many processes have been investigated for the manufacture, using the activities of specific cells, of substances which are impossible or difficult to synthesize purely artificially. Examples of such substances include interferon, hormones and lymphokines.

Cell culture is usually carried out by implanting or inoculating cells onto a culture bed, placing the bed in a culture medium and then culturing the cells under conditions appropriate to the particular cell strains. However, there are various limitations which make such cell culture rather difficult. One serious obstacle is the problem of the bed employed for the culture. In particular, since anchorage-dependent cells (such as normal diploid cell strains) adhere to a culture bed and proliferate only in a monolayer, the yield of cells after the culture will depend greatly on the nature of the culture bed used and, in particular, on the adhesiveness of the cells to the bed. Until now, high molecular weight compounds of polysaccharides or certain synthetic polymers have been used as, or as components of, the culture bed. However, these substances do not necessarily give satisfactory results and alternative materials are, accordingly, desirable.

Chemical Abstracts, *84*, p57, No. 136782y (1976) and Chemical Abstracts, *81*, p4 No. 64028v (1974) both disclosed sulphonated polystyrene surfaces which may be used for cell culture.

We have now discovered that the use of certain sulphonated polymers, at least for a surface of the culture bed, enables cells to be cultured efficiently and with a high yield and that such polymers have good adhesion to adhesion-dependent cells.

Accordingly, the present invention consists in a cell-culture bed, at least a surface of which comprises a copolymer of styrene and a sulphonated styrene and/or comprises a sulphonated polystyrene, characterized in that the degree of sulphonation of said copolymer or of said polystyrene is 50 mole % or less.

The invention further consists in a method of culturing cells on a culture bed in a culture medium for said cells, at least a surface of said bed comprising a copolymer of styrene and a sulphonated styrene and/or comprising a sulphonated polystyrene, characterized in that the degree of sulphonation of said copolymer or of said polystyrene is 50 mole % or less.

Hereafter, for conciseness, the styrene/sulphonated styrene copolymer and the sulphonated polystyrene are collectively referred to as the "sulphonated polymer", which term means the styrene/sulphonated styrene copolymer or the sulphonated polystyrene or a mixture thereof. In accordance with the invention, the culture bed employed may consist of the sulphonated polymer or may consist of a substrate which supports a surface layer composed of the sulphonated polymer.

The invention is further described with reference to the accompanying drawings, in which:

Figures 1 and 2 are sectional views showing different embodiments of cell-culture bed in accordance with the present invention; and

Figures 3 to 5 are graphs showing the results of cell culture experiments which were conducted employing the cell-culture beds of the present invention, and which are described in more detail in the following Examples.

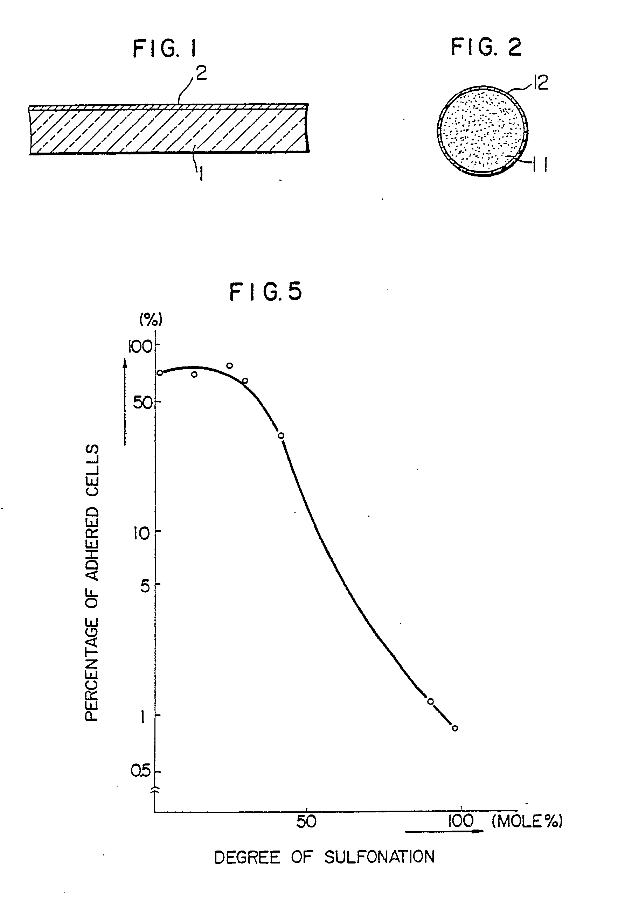

The embodiment of the invention shown in Figure 1 is a culture bed in the form of a plate and the Figure shows a cross-sectional view through the plate. The plate consists of a substrate 1, which may be made from a wide variety of materials, although it is preferably made of glass; the plate itself is preferably a Petri dish, a bottle or the like. On the surface of the substrate 1 is formed a layer 2 composed of the sulphonated polymer and it is this surface layer 2 which serves as the culture bed for cell culture. Formation of the surface layer 2 can be carried out by any convenient method. Most simply, a solution of the sulphonated polymer in a volatile solvent is dropped on the surface of the substrate 1 and spread over the surface, after which the solvent is removed by evaporation, leaving a film of the sulphonated polymer.

Alternatively, the culture bed may be formed from a plurality of particles, preferably fine particles, such as those illustrated in Figure 2, which shows a cross-section through such a particle. The particle shown in this Figure comprises a substrate 11 having a surface coating 12 of the sulphonated polymer. A wide variety of materials can be employed to form the substrate 11, although the material chosen is preferably not one which is toxic to the cells to be cultured and which would thus tend to hinder their proliferation. Suitable materials include acrylamide polymers and high molecular weight compounds of polysaccharides, both of which have hitherto been used as materials for preparing fine particles for use as a culture bed. The particle diameter of the substrate 11 is preferably within the range of from 10 μm to 2 mm, more preferably from 50 μm to 1 mm. The coating 12 can be formed on the substrate 11 by various methods. One simple method comprises immersing the fine particles of substrate 11 in a solution of the sulphonated polymer and then drying the substrate; alternatively, the fine particles of substrate 11 may be immersed in a bath containing the sulphonated polymer in molten form.

The cell-culture bed of the invention as shown in Figure 1, which consists of a plate substrate 1 having a surface layer 2 formed thereon, can conveniently be used when the culture is conducted on a laboratory or similar scale. On the other hand, the cell-culture bed of the invention shown in Figure 2 and consisting of fine particles of substrate 11 having a surface coating 12 has a remarkably large surface area relative to its volume and consequently provides a large effective area for cell culture; accordingly, such a cell-culture bed is extremely effective for the culture of anchorage-dependent cells, which proliferate in monolayers, when carried out on an industrial scale.

The cell-culture bed of the present invention is not limited to the particular shapes shown in Figures 1 and 2, but can have any shape appropriate to its particular intended use. Whatever the shape of the cell-culture bed, the thickness of the sulphonated polymer forming its surface can be selected freely and even a surface layer in the form of a monomolecular film is effective. In fact, there is no great difference in function or effect between the case where the surface layer is of monomolecular thickness and the cases where the surface layer is very thick or where the whole bed is composed of the sulphonated polymer. It is preferred that the entire surface of the substrate should be coated with a layer of the sulphonated polymer; however, even if the sulphonated polymer does not cover the entire surface of the substrate, the resulting bed is still, of course, effective.

Apart from the structure described above, in which the surface of the culture bed is composed of the sulphonated polymer, it is also possible to produce a culture bed in the form of a plate or of fine particles consisting only of the sulphonated polymer.

The copolymer of styrene and a sulphonated styrene employed in the present invention may be prepared by copolymerizing styrene with the sulphonated styrene in accordance with conventional polymerization procedures. For example, a polymerization catalyst may be added to a mixed solution of styrene and the sulphonated styrene and the resulting mixture then heated to effect polymerization. By altering the proportion of styrene to sulphonated styrene, copolymers having various sulphonic acid group contents can be obtained. The sulphonic acid group content of the copolymer is hereinafter referred to as the "degree of sulphonation".

Sulphonated polystyrenes can be obtained by contacting a polystyrene (obtained by the conventional polymerization of styrene) with a sulphonating agent, in accordance with various known methods. For example, a preferred sulphonated polystyrene can be produced by the following method: a suitable catalyst (such as triethyl phosphate) is dissolved in dichloroethane; in the resulting solution is dissolved a polystyrene, to give a polystyrene solution; this polystyrene solution is maintained at about −30°C, whilst liquid γ-type anhydrous sulphuric acid, also maintained at −30°C, is added thereto; the mixture is then allowed to stand and its temperature to rise to ambient, resulting in sulphonation of the polystyrene to give a sulphonated polystyrene. The quantity of dichloroethane used is preferably about 10 ml per 1 g of polystyrene. The molar ratio of anhydrous sulphuric acid to triethyl phosphate is preferably within the range from 1:1 to 3:1, and the molar ratio of anhydrous sulphuric acid to polystyrene is preferably within the range from 0.14:1 to 5:1. By altering the proportion of anhydrous sulphuric acid to polystyrene, the degree of sulphonation of the sulphonated polystyrene obtained can be controlled.

Also, by allowing a shaped article having a polystyrene surface layer to contact concentrated sulphuric acid, fuming sulphuric acid or chlorosulphonic acid, there can be obtained a culture bed having a surface layer of sulphonated polystyrene. In this case, the degree of sulphonation can be adequately controlled by adjusting the contact temperature, contact time and other reaction conditions. The degree of sulphonation of the surface of the cell-culture bed of this invention thus obtained is usually 0.5 mole% or less.

The polystyrene-reduced number average molecular weight of the sulphonated polymer employed in this invention is preferably at least 10,000, more preferably from 50,000 to 500,000.

In this invention, the degree of sulphonation may be calculated by taking the case in which one sulphur atom (from a sulphonic acid group) is present per 8 carbon atoms of styrene as a degree of sulphonation of 100 mole%. The degree of sulphonation of the sulphonated polymer employed in the invention is 50 mole% or less.

The cell culture method of the present invention may be carried out under the same conditions as are employed when using conventional cell culture beds. For example, cells can be cultured by inoculating cells into the cell-structure bed of the invention and then contacting the bed with a culture medium. The precise culture conditions will, of course, vary depending upon the optimum growth conditions for the particular cells employed.

In general, however, the culture temperature is usually a value from 25°C to 45°C. The culture medium is preferably a medium containing bovine serum or fetal bovine serum in an amount of from 2 to 20% by weight or a medium containing lactalbumin hydrolyzate and from 2 to 20% by weight of bovine serum of fetal bovine serum. Such culture media are usually renewed every 1 to 4 days. During the culture, oxygen or oxygen and carbon dioxide will normally be supplied to the culture medium.

The time required for the cultivation may vary, depending upon the kinds of cells, the nature of the culture medium and other factors. However, usually a period of from several hours to several tens of hours is required. Upon completion of the cultivation, the culture medium is preferably replaced by a phosphate buffered saline (PBS) (−) solution (free from $Ca^{++}$), after which there is added a solution which has the effect of isolating the cells from the culture bed, for example a 0.25% by weight trypsin solution or a pronase EDTA solution. When the grown cells have been detached from the culture bed, they may be collected by various conventional solid-liquid separation techniques, such as filtration or centrifugation.

As will be seen from the following Examples, since the surface of the cell-culture bed of the present invention is composed of a sulphonated polymer, the bed has a strong adhesion to the cells, which enables proliferation of the cells to proceed smoothly and, accordingly, cultivation of cells, particularly anchorage-dependent cells which proliferate in monolayers, can be performed with a high degree of efficiency. When the cell-culture bed of the present invention is in the form of fine particles, a large area can be provided for the cell culture, and this not only enables the cultivation to be conducted efficiently but also enables extremely high yields to be obtained and, accordingly, makes possible cell cultivation on an industrial scale.

These advantages can be obtained when the cell-culture bed and cell-culture method of the present invention are applied to the cultivation of various types of cells and the particular kinds of cell or cell strain are not critical. Examples of cells which may be used include Chinese hamster lung cells, human embryo lung cells, human cells derived from cancer of the cervix uteri, diploid human fibroblasts, African green monkey kidney cells, primary monkey kidney cells, baby hamster kidney cells, human embryo kidney cells, chimpanzee hepar fibroblast cells, human foreskin cells and chicken foetus fibroblast cells.

It would appear that one reason for the advantages of the use of a sulphonated polymer in accordance with the present invention is that the cell-culture bed of the invention has hydrophilic properties because of the sulphonic acid groups of the sulphonated polymer. However, the mere possession of hydrophilic properties by the polystyrene does not necessarily mean that it provides a good material for a cell-culture bed. This can be seen from the fact that increasing the sulphonic acid groups in the polystyrene does not necessarily enhance the performance of the cell-culture bed and also from the fact that the introduction of carboxy groups into the polystyrene in place of the sulphonic acid groups (which equally gives rise to hydrophilic properties) does not provide a satisfactory material for the cell-culture bed.

The invention is further illustrated by the following Examples.

Example 1

A total of six kinds of styrene/sulphonated styrene copolymers, differing from one another in their content of sulphonic acid groups, were prepared by copolymerizing styrene and p-sulphonated styrene, so that the molar percentages of the sulphonated styrene in the copolymers were 0.4, 1.8, 2.5, 5.0, 6.5 and 9.9. Each of these copolymers was then dissolved in benzene to a concentration of 5 mg/ml, except that the copolymer containing 9.9 mole% of p-sulphonated styrene was dissolved in a 9:1 by volume mixture of benzene and ethanol. Each of the resulting solutions was dropped onto and spread on a 35 mm diameter Petri dish, and the dish was dried under vacuum at 100°C for 24 hours, to form a thin film of the copolymer on the dish. There was thus obtained a total of 6 different kinds of cell-culture bed in accordance with the present invention.

Each of these beds was sterilized by heat for 24 hours. Into each bed was then inoculated free cells of cells strain V—79 (from the lungs of Chinese hamsters), using a PBS (−) solution containing 0.25% by weight of trypsin. The cell strains were then cultured using a culture medium containing 10% of fetal bovine serum at 37°C in an incubator whose atmosphere was 95% by volume air and 5% by volume carbon dioxide.

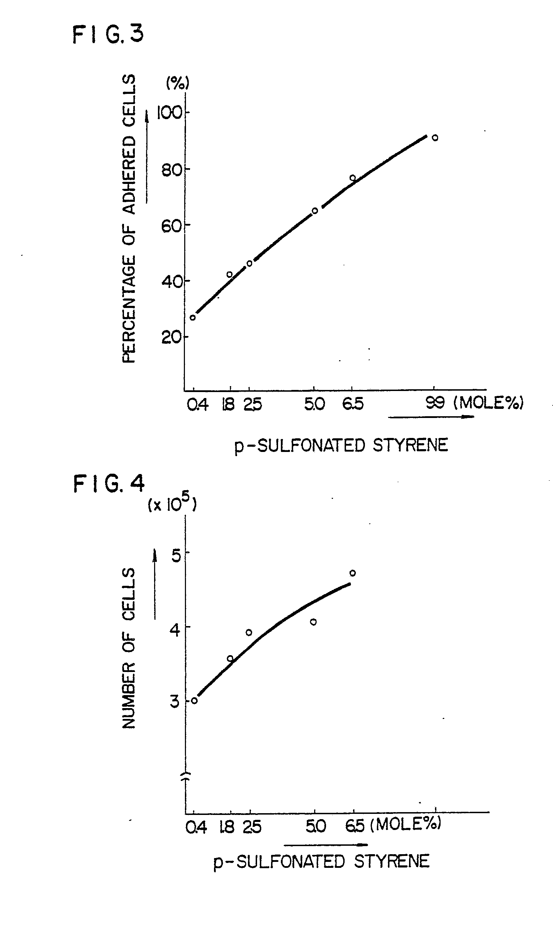

After cultivation for 120 minutes, the dishes were removed from the incubator. The culture medium was removed from each dish and the dish was washed with a PBS (−) solution. 1 ml of a pronase EDTA solution was then added to each dish, to detach cells which had adhered to the surface of the culture bed. The number of cells detached from each culture bed was measured by means of a hemacytometer. The relationship between the content of sulphonated styrene in the copolymer and the percentage of adhered cells, based on the total number of cells, is shown in Figure 3. From this Figure, it is clear that a high proportion of cells adhere to the cell-culture beds of the invention and thus cell-culture can be carried out most efficiently. It is also clear that, as the proportion of sulphonated styrene increases, the percentage of cells adhering to the bed increases significantly.

Example 2

The procedure described in Example 1 was repeated, except that the cultivation time was 3 days, instead of 120 minutes. The relationship between the content (in mole%) of sulphonated styrene in the copolymer and the percentage of adhering cells is shown in Figure 4, from which it is clear that a high culture yield can be obtained using the cell-culture bed of the invention.

Example 3

1 g of a polystyrene having a number average molecular weight of 200,000 and triethyl phosphate (in amounts varying from 0.2 to 8 g, in separate experiments) were dissolved in 20 ml of dichloroethane. The resulting solution was maintained at −30°C and stirred, whilst liquid γ-type anhydrous sulphuric acid maintained at −30°C was dropped onto it. The amount of sulphuric acid varied, in separate experiments, from 0.007 to 3.5 g. After all of the sulphuric acid had been added, the temperature of the resulting mixture was allowed to rise, with stirring, to room temperature, after which stirring was continued for a further 1 hour. The mixture was then poured into ethanol, to recover a sulphonated polystyrene.

By following the above procedure, a total of 7 different kinds of sulphonated polystyrenes having sulphonation degrees of 1, 13, 25, 30, 42, 91 and 98 mole% (the last two being for comparison, outside the scope of the present invention) were produced. The procedure described in Example 1 was then repeated, using these sulphonated polystyrenes to produce cell-culture beds, and then these were used for cultivation of cells, as in Example 1. In the case of those sulphonated polystyrenes having a degree of sulphonation of 13 mole% or more, the mixture of benzene and ethanol was used as solvent in place of the benzene. The relationship between the degree of sulphonation of the polystyrene and the percentage of adhering cells, based on the total number of cells after 120 minutes cultivation, is shown in Figure 5.

It is clear from the results shown in Figure 5 that, when the sulphonated polystyrene had a degree of sulphonation of 30 mole% or less, the percentage of adhering cells is very high. On the other hand, if the degree of sulphonation is too high, then the proportion of cells adhering decreases. From this it can be seen that degree of sulphonation should be 50 mole% or less.

From the above Examples taken together, it can be seen that 50% or less of the total benzene rings of the styrene/sulphonated styrene copolymer or of the sulphonated polystyrene should have sulphonic acid groups and that incubation can be carried out most satisfactorily when the proportion of benzene rings having sulphonic acid groups is about 20% of the total.

Example 4

A solution of a sulphonated polystyrene having a degree of sulphonation of 25 mole% was prepared as in Example 3. In this solution were immersed fine particles composed of a crosslinked dextran having an average particle diameter of 70 μm; the particles were then removed and dried at 100°C to form a thin film of sulphonated polystyrene on the surface of the particles. A cell-culture bed was prepared from these particles, was sterilized by heat and then placed in an incubator. The same cells as were used in Example 1 were cultured on this bed for 3 days, using the same culture medium and the same atmospheric conditions as in Example 1.

This experiment was repeated, using the same particles of dextran, but without the coating of sulphonated polystyrene. The cell-culture bed of the invention gave a culture yield about 1.5 times that of the bed using the uncoated dextran particles.

Example 5

5 ml of concentrated sulphuric acid were placed in each of 4 Petri dishes, each dish having a diameter of 5 cm and being made from polystyrene. Two of the dishes were kept at 25°C for 1 hour, whilst the other two dishes were kept at 25°C for 7 hours. The dishes were then each thoroughly washed with water. The degrees of sulphonation of the surfaces of these dishes were 1.5 and 1.0 mole%, respectively, as measured by ESCA.

Each of the dishes was sterilized by ultraviolet radiation and the dishes were then used as cell-culture beds for the cultivation of HeLa S3 cell strains (from human uterine cancer cells) and V—79 cell strains; the culture media and conditions were as described in Example 1. The number of cells inoculated was about $1 \times 10^5$ for each dish. The results are shown in Table 1.

TABLE 1

| Degree of sulfonation (mole %) | Percentage of adhered cells after culturing 18 hr (%) | |
|---|---|---|
| | HeLa S3 | V—79 |
| 1.5 | 68 | 48 |
| 9 | 62 | 56 |
| 0 (control) | 15 | 0 |

| Number of cells cultured | |
| --- | --- |
| HeLa S3 (after culturing for 11 days) | V—79 (after culturing for 7 days) |
| $8 \times 10^5$ | $30 \times 10^6$ |
| $9 \times 10^5$ | $28 \times 10^6$ |
| No proliferation | 0 |

### Example 6

5 g of spherical polystyrene particles of particle diameter $250 \pm 50$ μm, produced by suspension polymerization, were added to 50 ml of concentrated sulphuric acid, and the resulting mixture was stirred for 30 minutes at 25°C and then well washed with water. In a separate experiment, the same procedure was repeated, except that the mixture was stirred for 1 hour at 25°C. The degrees of sulphonation of the surfaces of the polystyrene particles were 0.9 and 2.4 mole%, respectively, as measured by ESCA.

The sulphonated polystyrene particles were then sterilized by ultraviolet radiation, and 0.5 g of each type of sulphonated particles was placed in a separate incubator for use as cell-culture beds. HeLa S3 and V-79 cells were then cultivated on these beds under the same conditions as in Example 1. The number of cells inoculated was about $1 \times 10^5$ in each case. The results are shown in Table 2.

TABLE 2

| Degree of sulfonation (mole %) | Percentage of adhered cells after culturing for 18 hr (%) | |
| --- | --- | --- |
| | HeLa S3 | V—79 |
| 0.9 | 59 | 37 |
| 2.4 | 54 | 45 |
| 0 (control) | 3 | 0 |

| Number of cells cultured | |
|---|---|
| HeLa S3 (after culturing for 11 days) | V—79 (after culturing for 7 days) |
| $60 \times 10^6$ | $150 \times 10^7$ |
| $75 \times 10^6$ | $120 \times 10^7$ |
| No proliferation | 0 |

## Claims

1. A cell-culture bed, at least a surface of which comprises a copolymer of styrene and a sulphonated styrene and/or comprises a sulphonated polystyrene, characterized in that the degree of sulphonation of said copolymer or of said polystyrene is 50 mole% or less.

2. A bed according to Claim 1, in the form of a plate or of fine particles.

3. A method of culturing cells on a culture bed in a culture medium for said cells, at least a surface of said bed comprising a copolymer of styrene and a sulphonated styrene and/or comprising a sulphonated polystyrene, characterized in that the degree of sulphonation of said copolymer or of said polystyrene is 50 mole% or less.

4. A method according to Claim 3, in which said bed is in the form of a plate or of fine particles.

## Revendications

1. Lit de culture de cellules dont au moins une surface comprend un copolymère de styrène et d'un styrène sulfoné et/ou comprend un polystyrène sulfoné, caractérisé en ce que le degré de sulfonation de ce copolymère ou de ce polystyrène est de 50 moles % ou moins.

2. Lit selon la revendication 1, sous forme d'une plaque ou de fines particules.

3. Procédé de culture de cellules sur un lit de culture dans un milieu de culture pour lesdites cellules, au moins une surface de cel lit comprenant un copolymère de styrène et d'un styrène sulfoné et/ou comprenant un polystyrène sulfoné, caractérisé en ce que le degré de sulfonation de ce copolymère ou de ce polystyrène est de 50 moles % ou moins.

4. Procédé selon la revendication 3, dans lequel le lit est sous forme d'une plaque ou de fines particules.

## Patentansprüche

1. Bett zur Züchtung von Zellen, bei dem mindestens eine Fläche ein Copolymer von Styrol und einem sulfonierten Styrol und/oder ein sulfoniertes Polystyrol umfaßt, dadurch gekennzeichnet, daß der Sulfonierungsgrad des Copolymers oder des Polystyrols 50 Mol-% oder weniger beträgt.

2. Bett nach Anspruch 1 in Form einer Platte oder feiner Teilchen.

3. Verfahren zur Züchtung von Zellen in einem Kulturmedium für die Zellen unter Verwendung des Betts, bei dem mindestens eine Fläche ein Copolymer von Styrol und einem sulfonierten Styrol und/oder ein sulfoniertes Polystyrol umfaßt, dadurch gekennzeichnet, daß der Sulfonierungsgrad des Copolymers oder des Polystyrols 50 Mol-% oder weniger beträgt.

4. Verfahren nach Anspruch 3, dadurch gekennzeichnet, daß das Bett in Form einer Platte oder feiner Teilchen vorliegt.

FIG. 1

FIG. 2

FIG. 5

PERCENTAGE OF ADHERED CELLS

(%)

100

50

10

5

1

0.5

50          100   (MOLE %)

DEGREE OF SULFONATION

1

# F I G. 3

F I G. 3

(%)

PERCENTAGE OF ADHERED CELLS

100

80

60

40

20

0.4  1.8  2.5      5.0   6.5              9.9  (MOLE%)

p-SULFONATED STYRENE

# F I G. 4

(× $10^5$)

NUMBER OF CELLS

5

4

3

0.4  1.8  2.5      5.0   6.5  (MOLE%)

p-SULFONATED STYRENE